# EUROPEAN PATENT APPLICATION

(11) **EP 1 688 087 A1**
(43) Date of publication of application: **09.08.2006**
(21) Application number: 06002447.8
(22) Date of filing: 07.02.2006
(51) Int. Cl.: A61B 5/00

(54) **A single use lancet device**

(30) Priority: 07.02.2005 US 52738
(71) Applicant: Schraga, Steven, Surfside, FL 33154 (US)
(72) Inventor: Schraga, Steven, Surfside, FL 33154 (US)
(74) Representative: Wagner, Karl H.

(57) **Abstract**

A compact single use lancet device wherein a lancet is movably disposed. A driving element is further provided and is structured to move the lancet into a piercing orientation wherein a piercing tip protrudes from the device and can pierce a patient's skin. A retention assembly maintains the lancet in a tensioned, ready tc fire orientation and prevents both inward and outward movement of the lancet, until released by an actuator. The actuator is structured to engage a patient and be urged inwardly into the housing in order to bring about the release of the lancet from its tensioned, ready to fire orientation and result in driven movement of the lancet into the piercing orientation. A protective cover is also included to engage the lancet and shield a piercing tip thereof, as well as to shield the actuator and prevent inware movement of the actuator as a result of the protective covers engagement with the retained lancet.

## Description

### BACKGROUND OF THE INVENTION

### Field of the Invention

The present invention relates to a single use lancet device structured to safely and effectively provide for a single use, thereby preventing cross contamination, and which is configured to be pre-cocked when provided to a user so as to facilitate manufacture and subsequent use and so as to readily identify a used device by virtue of it no longer being in a cocked state. Moreover, the present single use lancet device is further configured to effectively protect and shield a piercing tip thereof prior to use, and to also significantly prevent inadvertent firing thereof, either by a user, packager, or during transit, thereby increasing sterility prior to use and limiting waste and loss as a result of inadvertent firing.

### DESCRIPTION OF THE RELATED ART

Lancets are commonly utilized instruments which are employed both in hospitals and other medical facilities, as well as by private individuals, such as diabetics, in order to prick or pierce a patient's skin, typically on a finger of a patient, thereby leading to the generation of a blood sample which can be collected for testing. Because of the wide spread use of such lancets, there are a variety of lancet devices which are available for utilizatior by patients and/or practitioners in a variety of different circumstances.

For example, a typical lancet may merely include a housing with a sharp piercing tip that is pushed into the patient's skin. More commonly, however, lancet devices, which house a piercing tip and/or a lancet, have been developed which effectively encase and fire the lancet into the patient's skin, thereby eliminating the need for the person taking the sample to actually push the lancet tip into the skin.

Within the various types of specialized lancet devices, one variety is typically configured for multiple and/or repeated uses, while another category is particularly configured for single use, after which the entire device is disposed of. Looking in particular to the single use, disposable lancet devices, such devices typically include a housing which contains and directs or drives a piercing tip into the patient's skin, and which is disposed o: along with the used lancet. Naturally, so to make such disposable devices cost effective for frequent use, such devices tend to be rather simplistic in nature providing only a sufficient mechanist for firing, and not overly complicating the design so as to minimize that cost.

While existing single use devices are generally effective for achieving the piercing of the skin required for effective operation, such single use, disposable devices typically do not incorporate a large number of safety features to ensure the safe transport, use and disposal of the device. For example, one primary area of safety which must be addressed with all lancet devices pertains to the purposeful and/or inadvertent reuse of a contaminated lancet. Unfortunately, most currently available single use lancet devices are configured such that after a use thereof has been achieved, it is possible for a patient to re-cock the device, thereby allowing for a subsequent, inappropriate use.

As a result, it would be highly beneficial to provide a single use lancet device which is substantially compact and disposable, can be manufactured in a substantially cost effective manner, and which nevertheless is substantially safe to utilize, affirmatively preventing re-use, once contaminated. Moreover, it would be beneficial to provide such a single sue device that minimizes the risk of user confusion relative to whether the lancet device has been used or not, user and which provides for safe and effective handling prior to use in a manner that will not result ir inadvertent firing of the lancet device.

### SUMMARY OF THE INVENTION

The present invention is directed towards a single use lancet device which is substantially compact and cost effective to produce, but which still provides significant safety features to prevent inadvertent use or re-use. Moreover, the present single use lancet device is configured to be provided to a user in a secure and sterile pre-cocked state, thereby allowing the user tc readily identify if the lance device has already been used as a result of its no longer being in its pre-cocked state, and significantly reducing the potential for malfunction or the set up requirements to a user, without risking inadvertent firing during handling and transit.

Specifically, the single use lancet device of the present invention includes a preferably compact, housing. Moreover, movable disposed within the housing is a lancet. The lancet, which at a minimum includes a sharp piercing tip, is structured to be moved, preferably rapidly, into a piercing orientation wherein the tip pierces a patient's skin, by a driving element. In order to prevent the movement into the piercing orientation until required, the present single use lancet device further includes a retentior assembly. In particular, the retention assembly maintains the lancet in a tensioned, ready to fire orientation until released. In order to provide for the safe and controlled release of the lancet from that tensioned, ready to fire orientation, an actuator is further provided. The actuator is structured to engage a patient, preferably in a vicinity of the area to be pierced by the piercing tip, and be urged inwardly into the housing by the patient until it brings about the release of the lancet resulting in driven movement of the lancet into the piercing orientation. Additionally, so as to achieve safe and sterile pre-use handling of the single use lancet device a protective cover is also provided. Specifically, the protective cover is structured to engage the lancet, shielding a piercing tip thereof, and to at least partially shield the actuator so as to prevent inward movement of the actuator.

These and other objects, features and advantages of the present invention will become more clear when the drawings as well as the detailed description are taken into consideration.

### BRIEF DESCRIPTION OF THE DRAWINGS

For a fuller understanding of the nature of the present invention, reference should be had to the following detailed description taken in connection with the accompanying drawings in which:

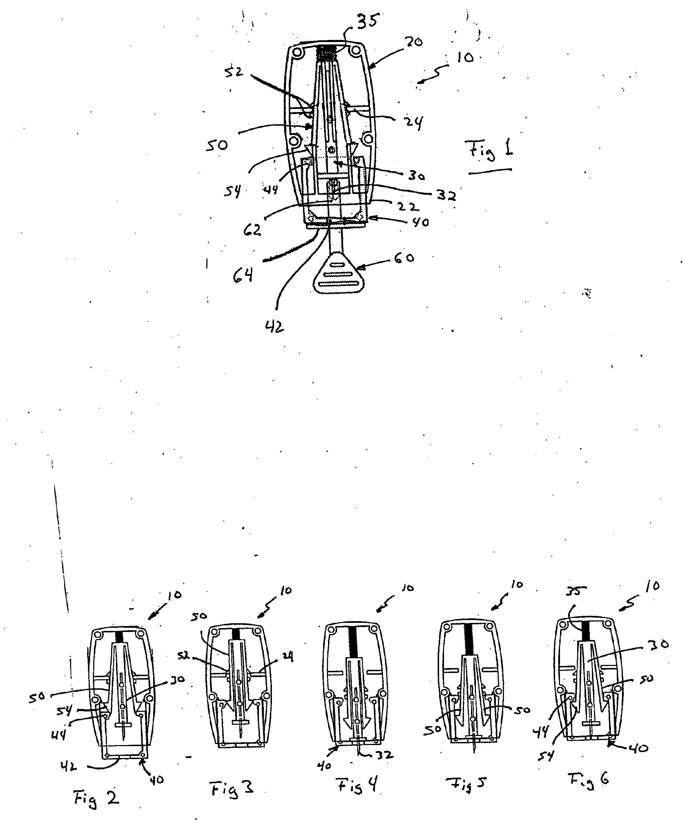

Figure 1 is a cross section of a preferred embodiment of the single use lancet device in a pre-cocked, covered orientation;
Figure 2 is a cross section of a preferred embodiment of the single use lancet device in a cocked, ready to use orientation;
Figure 3 is a cross section of a preferred embodiment of the single use lancet device upon release from it's tensioned, ready to fire orientation;
Figure 4 is a cross section of a preferred embodiment of the single use lancet device in a piercing orientation;
Figure 5 is a cross section of a preferred embodiment of the single use lancet device with the release elements in a relaxed, post fired orientation; and
Figure 6 is a cross section of a preferred embodiment of the single use lancet device in a post fired, at rest orientation wherein the spring has returned the lancet into a retracted, concealed post use position.

Like reference numerals refer to like parts throughout the several views of the drawings.

### DETAILED DESCRIPTION OF THE PREFERRED EMBODIMENT

As shown throughout the figures, the present invention is directed towards a single use lancet device, generally indicated as 10. In particular, the single use lancet device 10 of the present invention is configured to be substantially compact and easy to use, and includes safety features incorporated therein so as to minimize the risk that the lancet device 10 will be re-used after an initial use, and moreover which ensures that the lancet device can be safely handled prior to use without inadvertent firing thereof. Looking specifically to the preferred, illustrated embodiment of the single use lancet device 10, it includes a housing 20. The housing 20 may take on any of a number of different configurations, however, the housing 20 will preferably be substantially compact and easy to manipulate by an ultimate user. Moreover, although a variety of materials can be ultimately incorporated to form the housing 20, it may be preferred that the housing 20 be formed of a single or multiple part molded plastic ir order to facilitate cost effective manufacturing and disposal after a single use. The housing 20 preferably includes an open interior and a first end 22 which for ease of explanation can be termed the main face through which firing of the lancet device 10 takes place in order to pierce a patient's skin.

In order to ultimately pierce the patient's skin, a lancet 30 is movably disposed within the housing 20. The lancet 30 may take on any of a variety of configurations so long as it includes a piercing tip 32 which will ultimately pierce the patient's skin. In this regard, in some configurations the lancet 30 may include only an elongate metal shaft which terminates at the piercing tip 32, and/or may include a plastic body from which the piercing tip 32 extends. Regardless of the specific configuration of the lancet 30, however, when in use the lancet 30 will preferably be moved toward the first end 22 of the housing 20 until ultimately it reaches a piercing orientation wherein the piercing tip 32 is exposed and can penetrate the skin of a patient in order to cause bleeding for blood sampling purposes.

In order to effectively move the lancet 30 into its piercing orientation, a driving element is also provided. It is recognized that the driving element can take on any of a number of configurations, including a variety of different types of biasing elements, gear, wedge or direct motion type elements. In the illustrated embodiment, however, the driving element comprises a spring 35 disposed between the lancet 30 and the housing 20 which will drive or move the lancet into its piercing orientation, and will also preferably retract the lancet 30 into a concealed, safe orientation after use. Moreover, it is preferred that the single use lancet device 10 of the present invention be provided to an ultimate user in a pre-cocked and/or tensioned and ready to fire state, thereby eliminating the need for a user to take any affirmative steps in order to effectively move the lancet into its tensioned and ready to fire state, and so as to provide an added safety feature whereby a user will readily be able to identify that the single use lancet device 10 in their possession has already been fired if they do not receive it in a tensioned, ready to fire orientation. Accordingly when using the spring 35 as the driving element, the spring 35 will be pre-compressed when the single use lancet 10 is provided to a user.

In order to maintain the lancet 30 in that tensioned and ready to fire orientation until acted upon by the driving element 35, a retention assembly is further provided. Specifically, the retention assembly holds the lancet 30 in a desired location or orientation within the housing 20 until firing is dictated by a user. In this regard, it is also noted that a variety of different retention assemblies may also be incorporated so as to effectively hold the lancet 30 in its the tensioned ready to fire orientation relative to the housing, which in turn maintains the spring 35 compressed and under tension and ready to move the lancet 30 somewhat rapidly into its piercing orientation when the retention assembly releases the lancet 30.

In a preferred embodiment of the retention assembly, it is structured not only to prevent the lancet 30 from moving in an outward direction relative to the housing 20 and into the piercing orientation, but it is also configured so as to prevent inward movement of the lancet until released. Specifically, this restriction of inward movement of the lancet 30 until released provides an added safety feature to the single use lancet device 10 during handling prior to use, as will be described in greater detail subsequently.

Looking to the figures and the preferred embodiment of the retention assembly, it preferably comprises a pair of lock elements that are operatively associated between the lancet 30 and the housing 20 so as to engage one another and retain the lancet 30 in its tensioned, ready to fire orientation. As such, in order tc effectively disengage those lock elements from one another when use of the single use lancet device 10 is desired, a release element is further provided, the release element effectively disengaging the lock elements from one another and providing the freedom of movement that results in outward firing of the lancet 30 until the piercing tip 32 moves into its piercing orientation to penetrate the skin of a user. Looking further to the preferred retention assembly, the pair of lock elements preferably includes a male lock element and a female lock element disposed between the housing and the lancet. Moreover, in the illustrated embodiment the housing 20 includes one or more protruding elements 24 which effectively define the male lock elements. Conversely, operatively associated with the lancet 30 is the female lock element which may include a slot or indentation, but in the preferred embodiment includes a pair of ridges 52 which effectively receive the male lock element 24 therebetween and prevent the inward or outward movement of the lancet 30 when engaged with one another.

Although the female lock element 52 may be disposed directly on the main body of the lancet 30, with alternate release elements being provided, in the illustrated embodiment the female lock element 52 is disposed on the release element, which in turn is coupled with the lancet 30. Specifically, the release element preferably includes at least one, or as illustrated, a pair of biasable fingers 50 coupled with the main body of the lancet 30. In this regard, the release element 50 is structured to move towards and away from a central axis of the lancet 30 and to thereby effectively result in engagement and/or disengagement between the lock elements 24 and 52.

In order to result in ultimate firing of the single use lancet device 10 of the present invention, which results from a release of the lancet 30 from its tensioned and ready to fire orientation, an actuator 40 is further provided. Although a variety of different actuators, such as different types of triggers, etc. may be provided, in the preferred, illustrated embodiment the actuator 40 is structured to engage the patient's skin at the site of ultimate piercing, and indeed an opening 42 defined therein through which the piercing tip 32 of the lancet 30 will ultimately protrude ir order to pierce the patient's skin. In this regard, a surface of the actuator 40 is structured to be abutted against the patient's skin and upon being pushed by the patient will move into the housing 20 until it ultimately results in release of the lancet 30 from its tensioned and ready to fire orientation, and driven movement of the lancet 30 by the driving assembly 35 can be achieved. Accordingly, it is seen that the actuator 40 will ultimately engage the retention assembly that holds the lancet 30 in its tensioned and ready to fire orientation releasing the engagement it has with the housing 20. In the illustrated embodiment, the actuator 40 is structured to engage the release element(s) 50, causing movement thereof towards the central axis of the lancet 30 and thereby resulting in disengagement between the lock elements 24 and 52 once sufficient displacement thereof is achieved. Furthermore, in order to better facilitate this releasing movement of the release elements 50, each of the release elements 50 preferably includes a protrusion 54 disposed thereon. The protrusions 54 can take on any of a variety of configurations, however a generally angled, sloped configuration as illustrated in the figures is preferred so as to facilitate firing of the lancet device, allow for smooth passage of the lancet 30 into the piercing orientation, and so as to minimize the ability to reuse the lance device 10, as will be described. In particular, even though the actuator 40 may include a generally straight walled interio: configuration which engages the release elements 50, and in particular the protrusions 54 of the release elements 50, a pair of engagement elements 44 are preferably provided on the actuator 40. In particular, the engagement elements 44 preferably engage the protrusions 54 on the release elements 50 resulting in a gradual increase of movement of the release elements 50 towards one another as the actuator 40 is pushed inward and the engagement elements 44 ride up the preferably sloped faces of the protrusions 54. Again it is noted, however, that the sloped configuration is not necessary but is provided to facilitate a smoother actuation of the single use lancet device 10. Additionally, by inclusion of the engagement elements 40, subsequent to their passage over the protrusions 54 of the release elements 50, either as a result merely of inward movement of the actuator 40 or more likely as a result of sufficient compression of the release elements 50 tc result in a firing of the lancet 30, the engagement elements 44 will ultimately find themselves behind the protrusions 54, as seer in the figures. Accordingly, a general abutment therebetween is achieved and the actuator 40 can no longer be returned to a usable configuration and/or location relative to the lancet 30, and more specifically the release elements 50. From this, it is seen that once the lancet device 10 has been utilized an initial time with the lancet 30 moving into its piercing orientation, reuse of the device is substantially prevented as the actuator 40 is no longer properly positioned so as to effectively move the release elements 50 toward one another, and cannot be readily returned to that position under normal use conditions.

In order to provide for safe handling of the single use lancet device 10 of the present invention prior to use, the present invention further includes a protective cover 60. In particular, the protective cover 60 is structured to shield the piercing tip 32 of the lancet 30 until removed, thereby effectively maintaining a clean and sterile state of the piercing tip 32 until used. In this regard, the protective cover 60 may include an elongate stem 62 that extends through the opening 42 in the actuator 40 until it engages the lancet 30 and effectively shields the piercing tip 32. In this regard, it is noted that the stem 62 of the protective cover 60 may be molded with a body of the lancet 30 or may merely be structured so as to be press fit onto the lancet 30 over the piercing tip 32 in the shielding orientation so as to prevent inadvertent release or removal until desired by a user. In addition to the elongate stem, the protective cover 60 also preferably includes a generally rigid shielding panel 64. Specifically, the generally rigid shielding panel 64 is structured to shield the actuator 40 so as to prevent inadvertent engagement thereof, and as a result inadvertent firing of the lancet device 10. In the preferred embodiment, the shielding panel 64 is preferably disposed in at least partially, but preferably completely covering relation over a surface of the actuator 40 that is intended to contact the patient. Moreover, because the stem 62 of the protective cover 60 engages the lancet 30, and the retention assembly is structured to prevent inward movement of the lancet 30, the protective cover 60 is effectively prevented from moving inwardly into the housing 20, while the retention assembly is effectively engaged. Accordingly, the shielding panel 64 cannot move inward and generally prevents inward pushing of the actuator 40 as it blocks direct contact of the actuator 40 at its exterior surface. Therefore, the present lancet device 10 can be provided to ultimate users in its pre-cocked and ready to fire orientation with minimal risk of inadvertent firing, and more specifically, inadvertent firing that leads to the waste and/or loss of ar otherwise usable lancet device. Indeed, this effective configuration essentially solves a concern associated with making lancet devices single use and including specific structure that prevents reuse, while also providing these single use lancet devices 10 to users in an often preferred pre-cocked condition., as merchants and consumers need not worry about excessive waste on loss due to premature firing during transit or at the ultimate site of use.

Since many modifications, variations and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents.

Now that the invention has been described,

## Claims

1. A single use lancet device comprising:
a) a housing;
b) a lancet movably disposed in said housing;
c) a driving element structured to move said lancet into a piercing orientation;
d) a retention assembly structured to maintain said lancet in a tensioned, ready to fire orientation;
e) an actuator structured to engage a patient and be urged inwardly into said housing, said actuator further structured tc bring about a release of said lancet from said tensioned, ready tc fire orientation thereby resulting in driven movement of said lancet into said piercing orientation; and
f) a protective cover, said protective cover structured to engage said lancet and shield a piercing tip thereof, and to at least partially shield said actuator so as to prevent inward movement of said actuator.

2. A single use lancet device as recited in claim 1 wherein said actuator includes an opening defined therein through which said piercing tip of said lancet passes in order to contact and pierce the skin of the patient.

3. A single use lancet device as recited in claim 1 wherein said actuator is structured to engage and release said retention assembly.

4. A single use lancet device as recited in claim 3 wherein said retention assembly, when oriented to maintain said lancet in said tensioned, ready to fire orientation is structured to prevent both inward and outward movement of said lancet until released.

5. A single use lancet device as recited in claim 4 wherein said retention assembly comprises a pair of lock elements operatively associated with said lancet and said housing and structured tc engage one another to retain said lancet in said tensioned, ready to fire orientation, and a release element structured to disengage said lock elements from one another when engaged by said actuator.

6. A single use lancet device as recited in claim 5 wherein said pair of lock elements include a male lock element and a female lock element, engagement therebetween preventing inward and outward movement of said lancet.

7. A single use lancet device as recited in claim 6 wherein at least one of said lock elements is disposed on said release element, movement of said release element generally away from said other one of said lock elements by said actuator resulting in withdrawal of said male lock element from said female lock element and accordingly release of said lancet from said tensioned, ready to fire orientation.

8. A single use lancet device as recited in claim 7 wherein said release element is coupled to said lancet.

9. A single use lancet device as recited in claim 8 wherein said release element comprises a protrusion thereon structured to be engaged by said actuator upon inward movement thereof resulting in releasing movement of said release element.

10. A single use lancet device as recited in claim 9 wherein said protrusion on said release element is structured to engage said actuator subsequent to movement of said lancet into said piercing orientation in a manner which restricts withdrawal of said actuator from an inward position in said housing, thereby preventing subsequent actuating inward movement of said actuator after a single use of said lancet.

11. A single use lancet device as recited in claim 4 wherein saic protective cover engages said lancet and as a result of saic retention assembly preventing inward movement of said lancet, inward movement of said protective cover is also prevented.

12. A single use lancet device as recited in claim 11 wherein said protective cover includes a generally rigid shielding panel structured to extend at least partially over a surface of the actuator intended to contact the patient such that restriction of inward movement of said protective cover further results ir restriction of inward movement of said actuator.
